# EUROPÄISCHE PATENTSCHRIFT

(11) **EP 4 069 078 B1**
(45) Veröffentlichungstag und Bekanntmachung des Hinweises auf die Patenterteilung: **02.09.2026**
(21) Anmeldenummer: 20816920.1
(22) Anmeldetag: 27.11.2020
(51) Int. Cl.: A61B 5/107

(54) **SYSTEM UND VERFAHREN ZUR ERFASSUNG EINER OBERFLÄCHENFORM AM MENSCHLICHEN ODER TIERISCHEN KÖRPER**
DEVICE AND METHOD FOR DETECTING A SURFACE SHAPE ON THE HUMAN OR ANIMAL BODY
DISPOSITIF ET PROCÉDÉ DE DÉTECTION D'UNE FORME DE SURFACE SUR LE CORPS HUMAIN OU ANIMAL

(30) Priorität: 03.12.2019 CH 15322019
(43) Veröffentlichungstag der Anmeldung: 12.10.2022
(73) Patentinhaber: Bellwald Tec GmbH - NWS, 4132 Muttenz (CH)
(72) Erfinder: RUEPP, Thomas, 4132 Muttenz (CH); PASCAL, Joris, 4142 Münchenstein (CH); SCHUMACHER, Ralf, 4411 Seltisberg (CH)
(74) Vertreter: Braunpat AG
(86) Internationale Anmeldenummer: PCT/EP2020/083588
(87) Internationale Veröffentlichungsnummer: WO 2021/110547

(56) Entgegenhaltungen:
- CN-A- 105 877 753
- CN-A- 108 195 336
- DE-A1- 102006 004 514

## Beschreibung

Die Erfindung betrifft ein System und ein Verfahren zur Erfassung einer Oberflächenform am menschlichen oder tierischen Körper, insbesondere zur digitalen Erfassung einer dreidimensionalen Formgebung von Gliedmassen eines menschlichen oder tierischen Körpers.

Es besteht ein grosser und sehr unterschiedlicher Bedarf eine räumliche Formgebung von Oberflächen eines menschlichen oder auch tierischen Körpers erfassen zu können. So werden etwa Patienten mit Fehlbildungen oder Fehlhaltungen mit optischen Verfahren ausgemessen, um individuelle Behandlungslösungen wie Schienen, Korsette und dergleichen herstellen und anpassen zu können. Auch für die Herstellung von Prothesen ist es notwendig, eine dreidimensionale Form eines Extremitäten Stumpfes zu kennen, um einen optimalen Sitz der Prothese am Stumpf sicherstellen zu können.

In therapeutischen Bereichen, und hier insbesondere in der Orthopädie-Technik, besteht z. B. ein Bedarf einen Patientenscan durchführen zu können, während ein Therapeut oder Operateur eine therapeutische Handlung am Körper vornimmt oder während der Körper auf Stützeinrichtungen aufliegt. Etwa wird ein Scan erstellt während der Geradestellung einer spasmischen Deformität der unteren Extremitäten, um eine Schiene zum Festhalten einer geradgestellten Position zu erarbeiten.

Heutige Systeme verwenden z. B. Gipsabformungen mit späterer optischer Digitalisierung, oder aber es werden optische Digitalisiersysteme verwendet, die von aussen sichtbare Körperbereiche durch die Erfassung von an den Bereichen reflektiertem Licht abtasten.

Bei dem Einsatz von optischen Abtastverfahren zur Digitalisierung einer Körperoberfläche während einer therapeutischen Handlung am Körper kann jedoch eine Hand des Operateurs oder eine Stützeinrichtung in Konflikt mit der optischen Abtastung kommen. Die in der Orthopädietechnik verfügbaren digitalen Scan- und Modellierungseinrichtungen lassen diesen Missstand unbeachtet, wie z. B. die in DE 19822956 A1 beschriebene Vorrichtung zum Vermessen von Gliedmassen.

Es ist daher üblich Bereiche, die während einer Abtastung abgedeckt waren und daher nicht vermessen wurden, z. B. händisch nachzubearbeiten oder mittels Extrapolations- bzw. Rekonstruktionsverfahren zu ergänzen. Erst im Nachgang zur Messung können die erfassten Oberflächendaten durch aufwändige und unsichere Rekonstruktion von fehlenden Daten zu einem geschlossenen Oberflächenmodell aufbereitet werden, welches dann einer digitalen Fertigung von orthopädischen Hilfsmitteln zur Verfügung gestellt werden kann.

Weiter werden in der Orthopädie durchleuchtende bildgebende Verfahren, wie etwa Computertomographie oder Magnet-Resonanz-Tomographie, angewendet, um insbesondere die Formgebung innerer Körperbestandteile zu erfassen und abzubilden. Derartige Verfahren können aufgrund von Strahlenbelastung und hohen Feldstärken jedoch gesundheitsschädigend sein und liefern dennoch oftmals eine schlechte Datenqualität. Ferner sind sie umständlich und teuer in der Anwendung und erfordern einen hohen Energieaufwand.

Kleinere Vorrichtungen sind z. B. in WO 2005/013814 offenbart, in der eine optische Abtasteinrichtung zum Erfassen von Parametern innerer Körperteile, wie Organe, Muskeln, Fett oder anderem Gewebe, während einer Untersuchung der Körperteile durch einen Operateur gezeigt ist. Es können Anomalien der Körperteile, wie etwa Tumore, erfasst werden, nicht jedoch die äussere Formgebung von Köperoberflächen.

Ferner wurden für andere Gesundheits- und medizinische Bereiche unterschiedliche Abtast- und Datenerfassungsverfahren entwickelt, die jedoch für die dreidimensionale Erfassung von Körperoberflächen nicht geeignet sind. Insbesondere wurden Textilien mit integrierter Sensorik entwickelt, die z. B. in Form von Kleidungsstücken am Körper getragen werden können und unter der englischen Bezeichnung Smart Electro-Clothing Systems bekannt sind. Derartige Systeme können Biosignale erfassen, wie z. B. bioelektrische, biomagnetische oder biochemische Signale. Hierfür werden Sensoren unterschiedlicher Art in die Textilien integriert, wie z. B. Beschleunigungsmesser, Gyroskope und Magnetometer. Die Sensorik sendet die erfassten Signale an eine Verarbeitungseinheit zur Auswertung und Darstellung gesundheitsrelevanter Informationen.

Aus der WO 2014/204323 ist z. B. ein dehnbares Textilgewebe mit integrierten kapazitiven Sensoren bekannt, mit welchen eine Dehnung des Gewebes und somit eine Bewegung eines Körperteils, welches die Dehnung hervorruft, erfasst werden kann. Ferner sind z. B. Socken oder Handschuhe bekannt, welche grossflächige Sensorfelder mit elektrischen Sensoren aufweisen, um eine Druckanwendung und Druckverteilung an einer Fusssohle oder Handfläche zu messen.

Wie erwahnt, ist es mit derartigen Smart Electro-Clothing Systems jedoch nicht möglich, die dreiimensionale Gestalt einer Körperoberfläche zu erfassen.

Aus den Schriften CN 108 195 336 A, DE 10 2006 004514 A1 und CN 105 877 753 A sind Vorrichtungen bekannt, die Anordnungen verwenden um mit Hilfe von Magnetfeldsensoren die Oberflächenform eines beliebigen Körpers zu erfassen.

Es ist somit eine Aufgabe der vorliegenden Erfindung ein System und ein Verfahren zur Erfassung einer Oberflächenform am menschlichen oder tierischen Körper zu schaffen, welche diese Oberflächenform zuverlässig und vollständig erfassen können, die möglichst ohne Nachbearbeitung erfasster Oberflächendaten auskommen, die kosten- und energiesparend betrieben werden können und kein Risiko für den Körper darstellen.

Insbesondere soll die Erfindung ein System und ein Verfahren zur Erfassung einer dreidimensionalen Form einer Körperoberfläche bereitstellen, die eine sichere Aussage über einen therapeutischen Ansatz eines orthopädischen Hilfsmittels, wie etwa eine Orthesen Form, zulassen.

Diese Aufgabe wird erfindungsgemäss durch ein System nach Anspruch 1 und ein Verfahren nach 11 gelöst. Vorteilhafte Ausgestaltungen und unterschiedliche Ausführungsvarianten der Erfindung gehen aus den Unteransprüchen hervor.

Ein System zur Erfassung einer Oberflächenform einer menschlichen oder tierischen Körperoberfläche nach der vorliegenden Erfindung umfasst ein Sensorfeld mit einer Vielzahl von Sensoren, das dazu ausgebildet ist, die Körperoberfläche an einer Vielzahl von Oberflächenbereichen der Körperoberfläche abzutasten und eine Vielzahl von zugehörigen Oberflächensignalen zu generieren. Weiter weist das System eine Signalverarbeitungseinheit auf, die dazu ausgebildet ist, die Oberflächenform der Körperoberfläche aus der Vielzahl von Oberflächensignalen der Vielzahl von Sensoren zu bestimmen. Dabei sind die Sensoren des Sensorfelds jeweils wenigstens einem Oberflächenbereich zugeordnet und liefern ein diesem Bereich zugeordnetes Oberflächensignal. Aus der Gesamtheit an Oberflächensignalen kann somit die Oberflächenform der Körperoberfläche ermittelt werden.

Erfindungsgemäss weist das System einen Magnetfeldgenerator zur Erzeugung wenigstens eines Magnetfelds im Umkreis des Sensorfeldes auf. Somit liegt im Umfeld der Körperoberfläche, deren Form bestimmt werden soll, ein Magnetfeld an. Weiter ist das Sensorfeld aus einer Vielzahl von Magnetfeldsensoren gebildet, die relativ zueinander zur Auflage auf der Körperoberfläche angeordnet sind. Dabei ist die Vielzahl von Magnetfeldsensoren der Vielzahl von Oberflächenbereichen zugeordnet, so dass die Magnetfeldsensoren entlang der Körperoberfläche verteilbar sind. Die Magnetfeldsensoren sind dazu ausgebildet, als Oberflächensignal ein Lagesignal bezüglich ihrer Ausrichtung und Position im Magnetfeld zu generieren. Das Lagesignal eines Magnetfeldsensors stellt zumindest Informationen bezüglich der räumlichen Ausrichtung des Sensors im Magnetfeld zur Verfügung. Vorzugsweise umfasst das Lagesignal auch Informationen über den Ort eines Sensors im Magnetfeld. Weiter kann das Lagesignal Informationen über die Position des Sensors im Sensorfeld, bzw. relative zu den übrigen Sensoren, bereitstellen. Eine Position und Orientierung eines Oberflächenbereichs der Körperoberfläche wird somit durch einen zugehörigen Magnetfeldsensor repräsentiert. Ein Oberflächenbereich entspricht dabei zumindest einem Bereich der Körperoberfläche, auf dem der Magnetfeldsensor aufliegt. Der Oberflächenbereich kann sich aber auch darüber hinaus erstrecken bis dahin, wo ein benachbarter Oberflächenbereich beginnt. Die Lagesignale der Magnetfeldsensoren stellen somit ein Netzwerk von Oberflächensignalen zur Verfügung, welche die gesamte Körperoberfläche und ggf. eine gesamte Körperumfangsfläche abbilden können. Somit kann die Signalverarbeitungseinheit die Oberflächenform der Körperoberfläche aus der Vielzahl von Lagesignalen bestimmen und ein geschlossenes Oberflächenmodel bereitstellen.

Vorteilhaft nimmt die Signalverarbeitungseinheit eine Digitalisierung der Oberflächenform der Körperoberfläche vor, so dass ein digitaler Datensatz der erfassten Körperoberfläche vorliegt, der unmittelbar zur Behandlung eines Patienten oder zur Erarbeitung medizinischer Hilfsmittel, wie etwa einer Orthese, verwendet werden kann. Das System kann somit jegliche dreidimensionale Oberflächenform einer dreidimensionalen menschlichen oder tierischen Körperoberfläche erfassen. Vorteilhaft wird dabei eine geschlossene Körperumfangsfläche erfasst, wie sie beispielsweise bei Gliedmassen vorliegt.

Das Abtasten der Körperoberfläche durch das Sensorfeld erfolgt durch die Kopplung und Anregung der Magnetfeldsensoren durch das Magnetfeld des Magnetfeldgenerators. Ein breitflächiges Abtasten wird durch die Ausdehnung des Sensorfeldes über die Körperoberfläche ermöglicht.

Das Verfahren zur Erfassung einer Oberflächenform einer menschlichen oder tierischen Körperoberfläche nach der vorliegenden Erfindung verwendet vorzugsweise ein Erfassungssystem, wie es oben beschrieben ist. Bei dem erfindungsgemässen Verfahren wird die Vielzahl zueinander in einem Sensorfeld angeordneter Magnetfeldsensoren an der Vielzahl von Oberflächenbereichen der Körperoberfläche aufgelegt. Weiter wird mit dem Magnetfeldgenerator im Umkreis des Sensorfeldes ein Magnetfeld generiert. Dadurch wird mittels der Magnetfeldsensoren eine Vielzahl von zu den Oberflächenbereichen gehörenden Oberflächensignalen generiert, wobei die Oberflächensignale als Lagesignale bezüglich der Ausrichtung und Position der Magnetfeldsensoren im Magnetfeld vorliegen. Die Oberflächenform der Körperoberfläche wird dann mittels der Signalverarbeitungseinheit aus der Vielzahl von Lagesignalen der Magnetfeldsensoren bestimmt.

Das System und das Verfahren nach der vorliegenden Erfindung können die Oberflächenform zuverlässig und vollständig erfassen, da Behandlungen und Stützeinrichtungen problemlos über dem Sensorfeld auf die Körperoberfläche einwirken können ohne das Abtasten der Lagesignale zu behindern. Somit können auch Körperbereiche abgetastet werden, die von einer Hand oder einem Hilfsmittel während des Abtastens abgedeckt sind. Eine Nachbearbeitung erfasster Oberflächendaten z. B. zum Überbrücken von nicht abgetasteten Bereichen ist nicht erforderlich, wodurch eine präzise und detaillierte Erfassung der Körperoberfläche möglich ist.

In einer vorteilhaften Ausführungsvariante ist die Vielzahl von Magnetfeldsensoren in dem Sensorfeld in einem regelmässigen Raster angeordnet. Die Sensoren sind im Raster vorzugsweise in einem vorbestimmten Rasterabstand zueinander angeordnet. Der Rasterabstand kann z. B. entsprechend einer erforderlichen Genauigkeit der Oberflächenerfassung gewählt werden. Das Sensorfeld kann auch in Sektoren mit unterschiedlichen Rasterabständen unterteilt sein. Somit können z. B. in Bereichen der Körperoberfläche mit einer stark variierenden Oberflächenform kleinere Abstände gewählt werden, als in Bereichen mit einer eher einheitlichen Oberflächenform. Typischerweise liegt ein Abstand zwischen den Magnetfeldsensoren im Bereich von 1mm bis 500mm, vorzugsweise im Bereich von 5mm bis 50mm. Mit grossen Abständen von z. B. 50mm bis 500mm können grosse Körperoberflächen beispielsweise von grossen Tieren abgetastet werden. Auch wird bei der Verwendung grosser Abstände Material für die Sensoren und deren Verschaltung gespart.

Vorzugsweise ist ein Rasterabstand von Rasterreihen im Sensorfeld variabel. Das heisst, der Rasterabstand kann vergrössert oder verkleinert werden, je nach den Gegebenheiten einer Körperoberfläche oder einer Bewegung des Körpers. Auch kann sich der Rasterabstand des Sensorfeldes beim Auflegen der Magnetfeldsensoren auf die Körperoberfläche verändern, um ein akkurates Anbringen der Magnetfeldsensoren auf der Oberfläche zu ermöglichen.

Vorteilhaft sind dabei die Vielzahl von Magnetfeldsensoren des Sensorfeldes zueinander in einer deformierbaren Sensorebene angeordnet. Die Gesamtheit der nebeneinander angeordneten Magnetfeldsensoren bildet somit eine Ebene von Sensoren. Durch Bewegen benachbarter Sensoren relative zueinander ist diese Ebene deformierbar. Die Sensorebene ist damit in der Lage, sich an eine Körperoberfläche anzuschmiegen, so dass die Form der Ebene der Form der Körperoberfläche entspricht. Zum Aufspannen dieser Ebene können jeweils zwei oder mehr Sensoren z. B. durch flexible Verbindungmittel, wie etwa Fäden oder Kabel, miteinander verbunden werden. Auch ist es mögliche, die Sensoren durch elektrische Leitungen zum Abgreifen der Oberflächensignale miteinander zu verbinden. Die Sensorebene kann auch in sich geschlossen sein und eine Art Umfangsebene bilden.

In einer Ausführungsform sind die Vielzahl von Magnetfeldsensoren des Sensorfeldes oder ein Teil der Vielzahl von Magnetfeldsensoren in Serie miteinander verbunden. Das heisst, die Magnetfeldsensoren sind linear, bzw. in einer Reihe, mit einander verbunden. Ein Magnetfeldsensor ist dabei mit maximal zwei anderen Sensoren verbunden. Die in Serie miteinander verbundenen Magnetfeldsensoren bilden eine Art Sensorkette, die ausgelegt werden und die Sensorebene bilden kann. Dabei kann die Sensorkette z. B. geradlinig, in Kurven oder in Schleifen verlaufen. Alternativ können die Magnetfeldsensoren eine Art Sensornetz bilden, bei dem zumindest einige der Magnetfeldsensoren mit mindestens drei anderen Sensoren verbunden sind. Das Sensornetz kann dann die Sensorebene aufspannen.

In einer bevorzugten Ausführungsvariante ist die Vielzahl von Magnetfeldsensoren des Sensorfeldes in oder an einem Textilmaterial angebracht. Zur Erfassung der Form der Körperoberfläche wird das Textilmaterial mit den Magnetfeldsensoren derart auf einer Körperoberfläche aufgelegt, dass die Vielzahl von Magnetfeldsensoren an der zugeordneten Vielzahl von Oberflächenbereichen der Körperoberfläche zu liegen kommen. Dabei richten die Magnetfeldsensoren ihre Lage entsprechend einer Orientierung eines zugehörigen Oberflächenbereichs aus. Das Textilmaterial kann somit dem einfachen Montieren der Magnetfeldsensoren relativ zueinander und auch einem einfachen und angenehmen Auflegen der Sensoren auf der Körperoberfläche dienen. Durch die Flexibilität des Textilmaterials passt sich das Material der Körperoberfläche an und positioniert die Magnetfeldsensoren automatisch entsprechend der Oberflächenausrichtung auf der Oberfläche.

Vorzugsweise ist das Textilmaterial dehnbar ausgebildet. Somit kann das Sensorfeld über eine Körperoberfläche, insbesondere um eine Körperumfangsfläche, gespannt werden und wird dort sicher in Position gehalten. Weiter kann das Textilmaterial derart über der Körperoberfläche gedehnt werden, dass sich ein vorbestimmter Abstand der Magnetfeldsensoren zueinander ändert. Aus der Änderung des vorbestimmten Abstands kann wiederum auf die Form der Körperoberfläche geschlossen werden. Die Änderung kann somit Bestandteil des Lagesignals der Magnetfeldsensoren sein.

In einer einfachen Variante das System nach der vorliegenden Erfindung ist das Textilmaterial z. B. als Textiltuch mit den Magnetfeldsensoren ausgebildet, das auf oder um eine Körperoberfläche gelegt werden kann. Das Textilmaterial mit den Magnetfeldsensoren kann beispielsweise aber auch in einer Socke, einem Strumpf, Handschuh, Ärmel oder dergleichen verarbeitet sein, so dass der Patient das Textilmaterial über seine Gliedmassen anziehen kann. Alternativ kann das Textilmaterial auch in einem Handschuh verwendet werden, der von einem Operateur getragen wird. Der Operateur kann mit dem Handschuh eine zu vermessende Köperoberfläche des Patienten abtasten. Dabei legt der Operateur seine oder ihre Hand mit dem Handschuh auf die Körperoberfläche auf. Auch kann der Operateur mit dem Handschuh über die Körperoberfläche fahren.

Die Magnetfeldsensoren sind als dreidimensionale Magnetfeldsensoren ausgebildet. In einer Ausführungsform des Systems zur Erfassung einer Oberflächenform einer menschlichen oder tierischen Körperoberfläche nach der vorliegenden Erfindung sind die Magnetfeldsensoren als Hall Sensoren ausgebildet. Mit Hall Sensoren kann die magnetische Flussdichte des von dem Magnetfeldgenerator erzeugten Magnetfelds vermessen und daraus die Lage eines Sensors im Magnetfeld und relativ zu anderen Sensoren bestimmt werden.

Weiter können als Magnetfeldsensoren magnetoresistive (MR) Sensoren, bzw. Sensoren in Form eines elektronischen Kompass, verwendet werden, beispielsweise TMR-, AMR- oder GMR-Sensoren. Auch eine Mischung verschiedener Sensorarten ist denkbar.

In einer Ausgestaltung umfassen die Magnetfeldsensoren jeweils zwei oder mehr Sensoreinheiten, die für eine dreidimensionale Messung ausgebildet und arrangiert sind. Beispielsweise sind zwei oder mehr Sensoreinheiten vorgesehen, die zueinander senkrecht angeordnet sind. Die Magnetfeldsensoren erfassen die Feldvektorinformationen, die zu ihrer Orientierung und Position im Magnetfeld gehören.

Gemäss der vorliegenden Erfindung ist der Magnetfeldgenerator als dreiachsige Magnetfeldquelle ausgebildet ist. Als Magnetfeldquelle können z. B. Elektromagnete, eine Spulen oder bewegliche Permanentmagnete zum Einsatz kommen. Mit der dreiachsigen Magnetfeldquelle können drei sich überlagernde Magnetfelder erzeugt werden. Jedes der Magnetfelder kann von den Magnetfeldsensoren erfasst werden, so dass ein daraus resultierendes Lagesignal der Sensoren drei räumliche Komponenten umfasst, wodurch eine präzise Lageinformation für jeden Sensor erhältlich ist. Alternativ zu einer dreiachsigen Magnetfeldquelle können z. B. auch drei einzelne Magnetfeldquellen in einem Dreieck zueinander angeordnet werden. Die drei Quellen bilden ein überlagertes Gesamtmagnetfeld für die Messung mit den Magnetfeldsensoren.

Vorteilhaft wird von dem Magnetfeldgenerator ein Magnetfeld der Stärke 10nT, vorzugsweise 100nT bis 100mT im Umkreis des Sensorfelds generiert. In einer Variante des Erfassungssystems zum Abtasten an einem Unterarm wurden z. B. 2mT verwendet.

Im Vergleich zu herkömmlichen magnetischen Bildgebungsverfahren zur Erfassung von Oberflächenformen, wie z. B. der Magnet-Resonanz-Tomographie, ist das bei dem Verfahren nach der vorliegenden Erfindung verwendete Magnetfeld um einen Faktor 10 bis 30 Millionen kleiner. Das erfindungsgemässe Verfahren benötigt daher weniger Energie und ist kostengünstiger.

In einer weiteren Ausführungsform des Systems zur Erfassung einer Oberflächenform einer menschlichen oder tierischen Körperoberfläche nach der vorliegenden Erfindung weist das Sensorfeld eine weitere Vielzahl an Sensoren in Form von Druck- und/oder Temperatursensoren auf. Diese Sensoren sind dazu ausgebildet, ein Signal entsprechend einem Druck und/oder einer Temperatur an einer Vielzahl von Oberflächenbereichen der Körperoberfläche zu bestimmen. Dabei müssen die Oberflächenbereiche dieser Sensoren nicht mit den Bereichen übereinstimmen, welche den Magnetfeldsensoren zugeordnet sind. Ferner müssen für diese Sensoren nicht gleich viele Bereiche sein vorgesehen sein, wie für die Magnetfeldsensoren.

Vorteilhaft sind jedoch die Magnetfeldsensoren und die Druck- und/oder Temperatursensoren zu einem Sensorbauteil zusammengefasst und können gemeinsam im Sensorfeld und an den Magnetfeldsensoren zugeordneten Oberflächenbereichen vorgesehen werden. Mit den Druck- und/oder Temperatursensoren können Druck- und/oder Temperatursignale an den zugehörigen Oberflächenbereichen ermittelt werden. In Kombination mit den Lagesignalen der Magnetfeldsensoren kann somit eine räumliche Druck- und/oder Temperaturverteilung auf der Körperoberfläche bestimmt werden.

Mit dem System und dem Verfahren zur Erfassung einer Oberflächenform einer menschlichen oder tierischen Körperoberfläche nach der vorliegenden Erfindung kann somit aus der Vielzahl von Lagesignalen ein digitaler Datensatz einer Umfangsfläche der menschlichen oder tierischen Körperoberfläche erstellt werden. Die Feinheit des Datengitters liegt dabei beispielsweise im Bereich von 1mm bis 50cm bei grossen Tieren und im Bereich von 1mm bis 10cm beim Menschen. Das System ist aus einfachen Bauelementen aufgebaut und weist eine tragbare Grösse auf, so dass das Verfahren auch ambulant eingesetzt werden kann. Das Erfassungsverfahren ist einfach in der Anwendung, birgt aufgrund geringer Magnetfeldstärken keine gesundheitlichen Risiken und kann kostengünstig betrieben werden. Insbesondere bei der Konstruktion und Anpassung orthopädischer Hilfsmittel wie Schienen, Orthesen, Prothesen und dergleichen, ist es vorteilhaft, dass alle Bereiche einer Körperumfangsfläche abgetastet werden können und somit keine Nachbearbeitung der erfassten Datensätze erforderlich ist. Damit kann der Sitz und der Tragekomfort von Hilfsmitteln deutlich verbessert werden.

Die Erfindung wird im Folgenden anhand der Figuren dargestellt, die lediglich zur Erläuterung dienen und nicht einschränkend auszulegen sind. Aus den Figuren offenbar werdende Merkmale der Erfindung sollen einzeln und in jeder Kombination als zur Offenbarung der Erfindung gehörend betrachtet werden. Es zeigen:
Fig. 1 eine schematische Darstellung einer ersten Variante eines Sensorfelds eines Systems zur Erfassung einer Oberflächenform einer menschlichen oder tierischen Körperoberfläche nach der vorliegenden Erfindung,
Fig. 2 eine schematische Darstellung einer zweiten Variante eines Sensorfelds eines Systems zur Erfassung einer Oberflächenform einer menschlichen oder tierischen Körperoberfläche nach der vorliegenden Erfindung und
Fig. 3 ein Flussdiagram eines Verfahrens zur Erfassung einer Oberflächenform einer menschlichen oder tierischen Körperoberfläche nach der vorliegenden Erfindung.

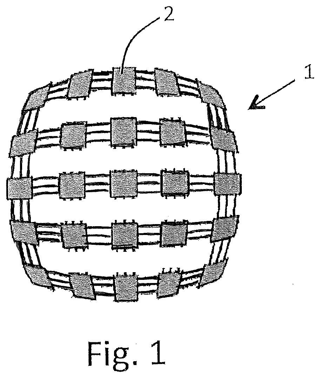

Wie in Figur 1 dargestellt, wird gemäss der Erfindung ein System zur Erfassung einer Oberflächenform einer menschlichen oder tierischen Körperoberfläche bereitgestellt, das ein Sensorfeld 1 mit einer Vielzahl von Magnetfeldsensoren 2 umfasst. Das Sensorfeld 1 ist dazu vorgesehen, eine dreidimensionale Körperoberfläche abzudecken, so dass die Körperoberfläche in eine Vielzahl von Oberflächenbereichen unterteilt ist. Jeder der Magnetfeldsensoren 2 tastet den ihm zugeordneten Oberflächenbereich ab und generiert ein Lagesignal mit Informationen zu seiner Lage auf der Körperoberfläche. Somit wird eine Vielzahl von Oberflächensignalen entsprechend der Vielzahl von Magnetfeldsensoren 2 und zugeordneten Oberflächenbereichen generiert.

Wie in Figur 1 ersichtlich ist, sind die Sensoreinheiten in einen Träger 4 in Form einer flexiblen, bzw. dehnbaren Leiterplatte (PCB) integriert. Die Magnetfeldsensoren 2 sind somit als dreiachsige Magnetfeldsensoren vorgesehen. Die Magnetfeldsensoren 2 erzeugen jeweils ein Lagesignal mit Informationen über die Orientierung des jeweiligen Sensors zur Körperoberfläche und vorteilhaft auch über die örtliche Lage der Magnetfeldsensoren zueinander.

Wie Figur 1 zu entnehmen ist, sind die Magnetfeldsensoren 2 in dem Sensorfeld 1 in einem Raster mit vorbestimmten regelmässigen Abständen zueinander angeordnet. Das Raster kann z. B. durch Anbringen der Magnetfeldsensoren 2 in einem Textilmaterial (nicht gezeigt) realisiert werden. In oder an dem Textilmaterial können auch Leiter (nicht gezeigt) zur Weiterleitung von digitalen Lagesignalen der Magnetfeldsensoren angebracht sein. Vorteilhaft sind die Sensoren seriell geschaltet, so dass nicht alle Sensoren miteinander verbunden sind und Material gespart werden kann.

In einer vorteilhaften Variante des Erfassungssystems nach der Erfindung bildet das Sensorfeld 1 eine deformierbare Sensorebene, wie in Figur 1 angedeutet ist. Hierfür kann z. B. das Textilmaterial, auf dem die Magnetfeldsensoren 2 angeordnet sind, flexibel und dehnbar sein, so dass die Orientierung der einzelnen Magnetfeldsensoren 2 zueinander und auch ihr Abstand variabel sind. Die Sensorebene kann sich somit einer beliebigen Form einer Körperoberfläche anpassen und die Magnetfeldsensoren entsprechend der Ausrichtung der jeweiligen Oberflächenbereiche ausrichten. Wie in Figur 1c angedeutet, ist die gezeigte Sensorebene z. B. nach aussen aus der Zeichenebene ausgebaucht.

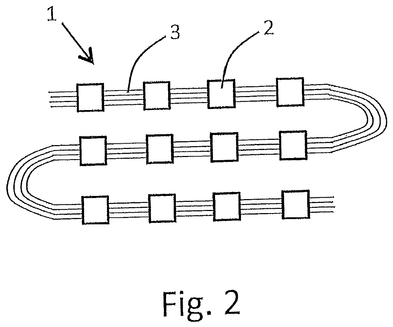

Figur 2 zeigt eine alternative Ausführungsform eines Systems zur Erfassung einer Oberflächenform einer menschlichen oder tierischen Körperoberfläche, bei dem die Magnetfeldsensoren 2 in Serie verlegt sind. Die Magnetfeldsensoren 2 sind mit vier Leitungen 3 in Reihe hintereinander geschaltet und bilden eine Art Sensorkette. Die Sensorkette kann wiederum auf einem Textilmaterial angebracht sein. Im gezeigten Beispiel ist die Sensorkette derart gebogen, dass sich drei im Wesentlichen parallele Reihen ergeben, die gemeinsam eine Sensorebene bilden. Auf diese Weise sind im System eine hohe Zahl an Magnetfeldsensoren und nur wenige Leitungen vorgesehen.

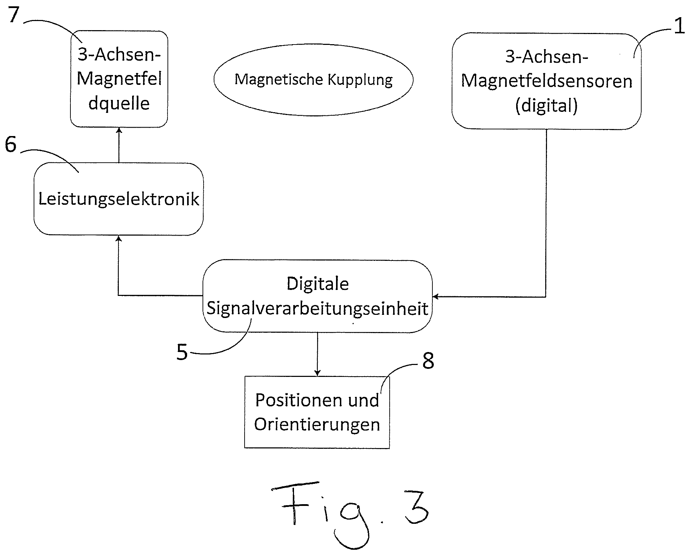

Figur 3 zeigt ein Diagramm zur Ausführung eines Verfahrens zur Erfassung einer Oberflächenform einer menschlichen oder tierischen Körperoberfläche nach der vorliegenden Erfindung. Eine Signalverarbeitungseinheit 5 sendet ein Aktivierungssignal an eine Leistungselektronik 6, um einen Magnetfeldgenerator 7 zu aktivieren. In dieser Variante ist der Magnetfeldgenerator 7 z. B. als eine drei-achsige Magnetfeldquelle gegeben, wie etwa in Form einer drei-achsigen Spule.

Der Magnetfeldgenerator 7 erzeugt ein Magnetfeld im Umkreis des Sensorfeldes 1. Somit umgibt das Magnetfeld die Vielzahl von Magnetfeldsensoren 2, die der Vielzahl von Oberflächenbereichen der Körperoberfläche angehören, und löst eine magnetische Anregung der Magnetfeldsensoren 2 aus. Die Magnetfeldsensoren 2 erzeugen ein Oberflächensignal in Antwort auf die Magnetfeldanregung entsprechend der magnetischen Feldstärke sowie ihrer Winkelorientierung und Position im Magnetfeld, welche wiederum von ihrer Orientierung gemäss der Körperoberfläche und ihrem Abstand zueinander abhängen. Diese Informationen können auf der Grundlage physikalischer Gesetze bezügliche der Abnahme der Magnetfeldstärke mit dem Abstand zur Magnetfeldquelle und der Winkelausrichtung im Magnetfeld ermittelt werden. Diese Informationen werden dann von der Vielzahl der Magnetfeldsensoren 2 als eine Vielzahl von Lagesignalen an die Signalverarbeitungseinheit 5 geschickt. Die Lagesignale bilden somit die Orientierung und die Ausdehnung der Oberflächenbereiche ab, so dass die Signalverarbeitungseinheit 5 daraus die Form der Körperoberfläche bestimmen und als digitalen Datensatz 8 der Oberflächenform ausgeben kann. Dieser digitale Datensatz kann z. B. direkt einem CAD-Programm zur Konstruktion eines orthopädischen Hilfsmittels zugeführt werden.

Obgleich das Erfassungsverfahren an einem Beispiel einer statischen Oberflächenformbestimmung erläutert wurde, ist es mit dem System und dem Verfahren nach der vorliegenden Erfindung durchaus möglich, eine dynamische Formbestimmung durchzuführen, bei der sich die Form einer Körperoberfläche z. B. entsprechend einer Bewegung eines Patienten währen der Abtastung ändert. Dabei ist lediglich darauf zu achten, dass sich der Patient innerhalb des von dem Magnetfeldgenerator erzeugten Magnetfeldes bewegt. Dadurch kann z. B. der Passsitz von orthopädischen Hilfsmitteln während einer Bewegung des Patienten ermittelt werden.

Das System und das Verfahren zur Erfassung einer Oberflächenform einer menschlichen oder tierischen Körperoberfläche nach der Erfindung hat somit weitreichende Einsatzmöglichkeiten und können gleichzeitig kostengünstig und energiesparen angewendet werden. Weiter ermöglichen sie eine hohe Genauigkeit der erfassten Oberflächenformen. Insbesondere kann ein geschlossenes Oberflächenmodel beispielsweise um eine Hand, einen Arm, einen Fuss und dergleichen erstellt werden, wie es in der Orthopädie-Technik vielfach benötigt wird.

### Bezugszeichenlegende

- 1: Sensorfeld
- 2: Magnetfeldsensoren
- 3: Leitungen
- 4: Träger
- 5: Signalverarbeitungseinheit
- 6: Leistungselektronik
- 7: Magnetfeldgenerator
- 8: digitaler Datensatz

## Patentansprüche

1. System zur Erfassung einer Oberflächenform einer menschlichen oder tierischen Körperoberfläche, welches
- ein Sensorfeld (1) mit einer Vielzahl von Sensoren umfasst, das dazu ausgebildet ist, die Körperoberfläche an einer Vielzahl von Oberflächenbereichen abzutasten und eine Vielzahl zugehöriger Oberflächensignale zu generieren, und
- eine Signalverarbeitungseinheit (5) aufweist, die dazu ausgebildet ist, die Oberflächenform der Körperoberfläche aus der Vielzahl von Oberflächensignalen der Vielzahl von Sensoren zu bestimmen, **gekennzeichnet durch**
- einen Magnetfeldgenerator (7), ausgebildet als dreiachsige Magnetfeldquelle, zur Erzeugung wenigstens eines Magnetfelds im Umkreis des Sensorfeldes (1) und
- eine Vielzahl von Magnetfeldsensoren (2), die das Sensorfeld (1) bilden und zueinander zur Auflage auf der Körperoberfläche angeordnet sind, wobei die Magnetfeldsensoren (2) dazu ausgebildet sind, als Oberflächensignal ein Lagesignal mit drei räumlichen Komponenten bezüglich ihrer Ausrichtung und Position im Magnetfeld zu generieren, welches Lagesignal eine Position und Orientierung eines Oberflächenbereichs der Körperoberfläche repräsentiert,
- so dass die Signalverarbeitungseinheit (5) die Oberflächenform der Körperoberfläche aus der Vielzahl von Lagesignalen bestimmt.

2. System nach Anspruch 1, **dadurch gekennzeichnet, dass** die Vielzahl von Magnetfeldsensoren (2) in dem Sensorfeld (1) in einem regelmässigen Raster angeordnet sind, wobei vorzugsweise ein Rasterabstand von Rasterreihen variabel ist.

3. System nach Anspruch 1 oder 2, **dadurch gekennzeichnet, dass** die Vielzahl von Magnetfeldsensoren (2) des Sensorfeldes (1) zueinander in einer deformierbaren Sensorebene angeordnet sind.

4. System nach einem der vorhergehenden Ansprüche, **dadurch gekennzeichnet, dass** zumindest ein Teil der Vielzahl von Magnetfeldsensoren (2) des Sensorfeldes (1) in Serie miteinander verbunden sind.

5. System nach einem der vorhergehenden Ansprüche, **dadurch gekennzeichnet, dass** die Vielzahl von Magnetfeldsensoren (2) des Sensorfeldes (1) in oder an einem Textilmaterial angebracht sind.

6. System nach Anspruch 5, **dadurch gekennzeichnet, dass** das Textilmaterial dehnbar ist.

7. System nach einem der vorhergehenden Ansprüche, **dadurch gekennzeichnet, dass** die Magnetfeldsensoren (2) als Hall oder als magnetoresistive Sensoren ausgebildet sind.

8. System nach einem der vorhergehenden Ansprüche, **dadurch gekennzeichnet, dass** die Magnetfeldsensoren (2) zwei oder mehr Sensoreinheiten umfassen, die für eine dreidimensionale Messung ausgebildet und arrangiert sind.

9. System nach einem der vorhergehenden Ansprüche, **dadurch gekennzeichnet, dass** der Magnetfeldgenerator (7) ein Magnetfeld der Stärke 10nT bis 100mT im Umkreis des Sensorfelds (1) generiert.

10. System nach einem der vorhergehenden Ansprüche, **dadurch gekennzeichnet, dass** das Sensorfeld (1) eine weitere Vielzahl von Druck- und/oder Temperatursensoren aufweist, die dazu ausgebildet sind, ein Signal entsprechend einem Druck und/oder einer Temperatur an einer Vielzahl von Oberflächenbereichen zu bestimmen.

11. Verfahren zur Erfassung einer Oberflächenform einer menschlichen oder tierischen Körperoberfläche, umfassend:
- Auflegen einer Vielzahl zueinander in einem Sensorfeld (1) angeordneten Magnetfeldsensoren (2) an einer Vielzahl von Oberflächenbereichen der Körperoberfläche,
- Generieren wenigstens eines drei-achsigen Magnetfeldes im Umkreis des Sensorfeldes (1) mittels eines Magnetfeldgenerators, ausgebildet als dreiachsige Magnetfeldquelle,
- Generieren einer Vielzahl von zu den Oberflächenbereichen gehörenden Oberflächensignalen mittels der Magnetfeldsensoren (2), wobei die Oberflächensignale als Lagesignale mit drei räumlichen Komponenten bezüglich der Ausrichtung und Position der Magnetfeldsensoren im Magnetfeld vorliegen, welche Lagesignale je eine Position und Orientierung eines Oberflächenbereichs der Körperoberfläche repräsentieren, und
- Bestimmen der Oberflächenform der Körperoberfläche aus der Vielzahl von Lagesignalen der Magnetfeldsensoren (2) mittels einer Signalverarbeitungseinheit (5).

12. Verfahren nach Anspruch 11, wobei die Magnetfeldsensoren (2) in oder an einem Textilmaterial angebracht sind, das derart auf einer Körperoberfläche aufgelegt wird, dass die Vielzahl von Magnetfeldsensoren (2) an einer Vielzahl von Oberflächenbereichen der Körperoberfläche zu liegen kommen, wobei die Magnetfeldsensoren (2) ihre Lage entsprechend einer Orientierung und Position eines zugehörigen Oberflächenbereichs ausrichten.

13. Verfahren nach Anspruch 12, wobei das Textilmaterial über der Körperoberfläche gedehnt wird, so dass sich ein vorbestimmter Abstand der Magnetfeldsensoren (2) zueinander ändert.

14. Verfahren nach einem der Ansprüche 11 bis 13, wobei aus der Vielzahl von Lagesignalen ein digitaler Datensatz (8) einer Umfangsfläche der menschlichen oder tierischen Körperoberfläche erstellt wird.

## Claims

1. System for detecting the surface shape of a human or animal body surface, which system
- comprises a sensor field (1) having a plurality of sensors which is designed to scan the body surface at a plurality of surface regions and to generate a plurality of associated surface signals, and
- has a signal processing unit (5) which is designed to determine the surface shape of the body surface from the plurality of surface signals from the plurality of sensors, **characterized by**
- a magnetic field generator (7), in the form of a three-axis magnetic field source, for generating at least one magnetic field in the vicinity of the sensor field (1) and
- a plurality of magnetic field sensors (2) which form the sensor field (1) and are arranged relative to each other in order to be placed on the body surface, wherein the magnetic field sensors (2) are designed to generate as a surface signal a positional signal having three spatial components with respect to their alignment and position in the magnetic field, which positional signal represents a position and orientation of a surface region of the body surface,
- such that the signal processing unit (5) determines the surface shape of the body surface from the plurality of positional signals.

2. System according to claim 1, **characterized in that** the plurality of magnetic field sensors (2 are arranged) in the sensor field (1) in a regular grid, wherein preferably a grid spacing of grid rows is variable.

3. System according to claim 1 or 2, **characterized in that** the plurality of magnetic field sensors (2) of the sensor field (1) are arranged relative to each other in a deformable sensor plane.

4. System according to any of the preceding claims, **characterized in that** at least some of the plurality of magnetic field sensors (2) of the sensor field (1) are interconnected in series.

5. System according to any of the preceding claims, **characterized in that** the plurality of magnetic field sensors (2) of the sensor field (1) are affixed in or to a textile material.

6. System according to claim 5, **characterized in that** the textile material is stretchable.

7. System according to any of the preceding claims, **characterized in that** the magnetic field sensors (2) are in the form of Hall sensors or of magnetoresistive sensors.

8. System according to any of the preceding claims, **characterized in that** the magnetic field sensors (2) comprise two or more sensor units which are designed and arranged to measure in three dimensions.

9. System according to any of the preceding claims, **characterized in that** the magnetic field generator (7) generates a magnetic field of the strength 10 nT to 100 mT in the vicinity of the sensor field (1).

10. System according to any of the preceding claims, **characterized in that** the sensor field (1) has a further plurality of pressure and/or temperature sensors which are designed to determine a signal corresponding to a pressure and/or a temperature at a plurality of surface regions.

11. Method for detecting the surface shape of a human or animal body surface, comprising:
- placing a plurality of magnetic field sensors (2), arranged relative to each other in a sensor field (1), on a plurality of surface regions of the body surface,
- generating at least one three-axis magnetic field in the vicinity of the sensor field (1) by means of a magnetic field generator, in the form of a three-axis magnetic field source,
- generating a plurality of surface signals belonging to the surface regions by means of the magnetic field sensors (2), wherein the surface signals are in the form of positional signals having three spatial components with respect to the alignment and position of the magnetic field sensors in the magnetic field, which positional signals each represent a position and orientation of a surface region of the body surface, and
- determining the surface shape of the body surface from the plurality of positional signals from the magnetic field sensors (2) by means of a signal processing unit (5).

12. Method according to claim 11, wherein the magnetic field sensors (2) are affixed in or to a textile material which is placed on a body surface in such a way that the plurality of magnetic field sensors (2) come to lie on a plurality of surface regions of the body surface, wherein the magnetic field sensors (2) align their position according to an orientation and position of an associated surface region.

13. Method according to claim 12, wherein the textile material is stretched over the body surface such that a predetermined distance of the magnetic field sensors (2) relative to each other changes.

14. Method according to any of claims 11 to 13, wherein a digital data set (8) of a circumferential surface of the human or animal body surface is created from the plurality of positional signals.

## Revendications

1. Système permettant de détecter une forme de surface d'une surface corporelle humaine ou animale, lequel
- comprend un réseau de capteurs (1) comportant une pluralité de capteurs qui est configuré pour balayer la surface corporelle au niveau d'une pluralité de zones de surface et pour générer une pluralité de signaux de surface associés, et
- présente une unité de traitement de signaux (5) qui est configurée pour déterminer la forme de surface de la surface corporelle à partir de la pluralité de signaux de surface de la pluralité de capteurs, **caractérisé par**
- un générateur de champ magnétique (7), conçu comme une source de champ magnétique à trois axes, permettant de générer au moins un champ magnétique dans le périmètre du réseau de capteurs (1) et
- une pluralité de capteurs de champ magnétique (2) qui forment le réseau de capteurs (1) et qui sont disposés les uns par rapport aux autres pour être appliqués sur la surface corporelle, dans lequel les capteurs de champ magnétique (2) sont configurés pour générer en tant que signal de surface un signal de position comportant trois composantes spatiales se rapportant à leur orientation et à leur position dans le champ magnétique, lequel signal de position représente une position et une orientation d'une zone de surface de la surface corporelle,
- de sorte que l'unité de traitement de signaux (5) détermine la forme de surface de la surface corporelle à partir de la pluralité de signaux de position.

2. Système selon la revendication 1, **caractérisé en ce que** la pluralité de capteurs de champ magnétique (2) sont disposés dans le réseau de capteurs (1) selon un quadrillage régulier, dans lequel, de préférence, un espacement de quadrillage de rangées de quadrillage est variable.

3. Système selon la revendication 1 ou 2, **caractérisé en ce que** la pluralité de capteurs de champ magnétique (2) du réseau de capteurs (1) sont disposés les uns par rapport aux autres dans un plan de capteurs déformable.

4. Système selon l'une des revendications précédentes, **caractérisé en ce qu'**au moins une partie de la pluralité de capteurs de champ magnétique (2) du réseau de capteurs (1) est connectée en série.

5. Système selon l'une des revendications précédentes,
**caractérisé en ce que** la pluralité de capteurs de champ magnétique (2) du réseau de capteurs (1) sont montés dans ou sur un matériau textile.

6. Système selon la revendication 5, **caractérisé en ce que** le matériau textile est extensible.

7. Système selon l'une des revendications précédentes,
**caractérisé en ce que** les capteurs de champ magnétique (2) sont conçus comme des capteurs à effet Hall ou comme des capteurs magnétorésistifs.

8. Système selon l'une des revendications précédentes, **caractérisé en ce que** les capteurs de champ magnétique (2) comprennent deux unités formant capteurs ou plus qui sont configurées et agencées pour une mesure tridimensionnelle.

9. Système selon l'une des revendications précédentes, **caractérisé en ce que** le générateur de champ magnétique (7) génère un champ magnétique d'une intensité allant de 10 nT à 100 mT dans le périmètre du réseau de capteurs (1).

10. Système selon l'une des revendications précédentes, **caractérisé en ce que** le réseau de capteurs (1) présente une autre pluralité de capteurs de pression et/ou de température qui sont configurés pour déterminer un signal correspondant à une pression et/ou à une température sur une pluralité de zones de surface.

11. Procédé permettant de détecter une forme de surface d'une surface corporelle humaine ou animale, comprenant :
- l'application d'une pluralité de capteurs de champ magnétique (2) disposés les uns par rapport aux autres dans un réseau de capteurs (1) sur une pluralité de zones de surface de la surface corporelle,
- la génération d'au moins un champ magnétique à trois axes dans le périmètre du réseau de capteurs (1) au moyen d'un générateur de champ magnétique, conçu comme une source de champ magnétique à trois axes,
- la génération d'une pluralité de signaux de surface appartenant aux zones de surface au moyen des capteurs de champ magnétique (2), dans lequel les signaux de surface sont présents en tant que signaux de position comportant trois composantes spatiales se rapportant à l'orientation et à la position des capteurs de champ magnétique dans le champ magnétique, lesquels signaux de position représentent respectivement une position et une orientation d'une zone de surface de la surface corporelle, et
- la détermination de la forme de surface de la surface corporelle à partir de la pluralité de signaux de position des capteurs de champ magnétique (2) au moyen d'une unité de traitement de signaux (5).

12. Procédé selon la revendication 11, dans lequel les capteurs de champ magnétique (2) sont montés dans ou sur un matériau textile qui est appliqué sur une surface corporelle de telle sorte que la pluralité de capteurs de champ magnétique (2) viennent se placer sur une pluralité de zones de surface de la surface corporelle, dans lequel les capteurs de champ magnétique (2) orientent leur position selon une orientation et une position d'une zone de surface associée.

13. Procédé selon la revendication 12, dans lequel le matériau textile est étiré sur la surface corporelle, de sorte qu'une distance prédéterminée des capteurs de champ magnétique (2) les uns par rapport aux autres varie.

14. Procédé selon l'une des revendications 11 à 13, dans lequel un ensemble de données numériques (8) d'une surface périphérique de la surface corporelle humaine ou animale est créé à partir de la pluralité de signaux de position.
